# EUROPÄISCHE PATENTSCHRIFT

(11) **EP 3 595 608 B1**
(45) Veröffentlichungstag und Bekanntmachung des Hinweises auf die Patenterteilung: **28.06.2023**
(21) Anmeldenummer: 18723778.9
(22) Anmeldetag: 03.05.2018
(51) Int. Cl.: A61B 90/50, A61G 13/10, A61G 13/12

(54) **MEDIZINISCHER HALTEARM MIT ARRETIERBAREM DREHGELENK**
MEDICAL HOLDING ARM WITH LOCKABLE ROTARY JOINT
BRAS DE SUPPORT MÉDICAL AVEC PIVOT VERROUILLABLE

(30) Priorität: 17.05.2017 DE 102017110718
(43) Veröffentlichungstag der Anmeldung: 22.01.2020
(73) Patentinhaber: Maquet GmbH, 76437 Rastatt (DE)
(72) Erfinder: KOCH, Guido, 76149 Karlsruhe (DE)
(74) Vertreter: Zacco GmbH
(86) Internationale Anmeldenummer: PCT/EP2018/061345
(87) Internationale Veröffentlichungsnummer: WO 2018/210576

(56) Entgegenhaltungen:
- US-A1- 2004 133 979
- US-A1- 2007 265 635
- US-A1- 2013 191 994

## Beschreibung

### Hintergrund der Erfindung

Die vorliegende Erfindung betrifft einen medizinischen Haltearm gemäß dem Oberbegriff des Anspruchs 1.

Ein solcher Haltearm ist bekannt. Er wird vom Unternehmen Mizuho OSI als Teil eines Operationstischs angeboten. Der Operationstisch wird unter der Marke ProFx vertrieben. Eine Präsentation des Operationstischs ProFx findet sich im Internet.

Dieser bekannte Haltearm ist über ein Kugelgelenk mit Kugel und Kugelpfanne an den Operationstisch angebunden. Der Haltearm kann dank des Kugelgelenks in verschiedene Richtungen gedreht werden. So kann die Chirurgin die Position eines Patientenbeins, welches am Haltearm befestigt ist, in die für den Eingriff optimale Stellung bringen. Wenn das Patientenbein richtig ausgerichtet ist, lässt sich der Haltearm über einen Klemmmechanismus arretieren. Hierzu wird die Kugelpfanne mechanisch auf der Kugel verspannt. Genauer gesagt wird dabei ein am Ende des Haltearms befindlicher Drehhebel umgelegt, der über eine innerhalb des Holms angeordnete Torsionswelle auf die Kugelpfanne einwirkt.

Dieser bekannte Haltearm hat insbesondere die folgenden Nachteile:
- Der Anwender muss mit dem Hebel eine hohe Schließkraft aufbringen. Zudem hat er nur einen festgelegten Verstellweg für den Hebel zur Verfügung und muss sich darauf verlassen, dass die über den Verstellweg zwischen Kugelpfanne und Kugel erzeugte Vorspannung ausreicht und der Haltearm somit sicher fixiert ist;
- Kugel und Kugelpfanne laufen trocken mit hohem Reibwert aufeinander. Dementsprechend verschleißt im Einsatz durch die Drehungen des Haltearms mindestens einer der Reibpartner;
- Wegen des Abriebs der Reibpartner ist ein regelmäßiges Nachstellen des Klemmmechanismus erforderlich, damit der Drehhebel sich immer im gleichen Bereich bewegt;
- Die frei liegende Kugeloberfläche verschmutzt und der Schmutz kann in das Gelenk gefördert werden, was auf Dauer zu einem Ausfall des Kugelgelenks führen kann;
- Da der Anwender im unverriegelten Zustand die Last des Haltearms mit einer Hand aufnehmen und mit der anderen Hand den Schließvorgang mit nicht unerheblichem Kraftaufwand ausführen muss, ergibt sich die Gefahr, dass der Haltearm und somit das Patientenbein ungewollt absinken, bevor der Haltearm sicher arretiert ist.

Die US 2013/191994 A1 beschreibt einen Operationstisch, der über zwei medizinische Haltearme verfügt.

Die US 2004/133979 A1 betrifft einen weiteren Operationstisch mit zwei medizinischen Haltearmen.

Der Fachfrau ist ein weiterer Haltearm bekannt, der in der WO 2007/080454 A2 beschrieben ist. Dieser bekannte Haltearm wird von dem Unternehmen Smith & Nephew als Teil eines Hüftpositioniersystems vertrieben. Eine Beschreibung dieses Systems findet man im Internet.

Auch dieser Haltearm kann über ein verriegelbares Kugelgelenk gedreht werden, siehe die Figuren 5 und 6 der WO 2007/080454 A2. Die Arretierung und die Freigabe des Kugelgelenks erfolgt über einen am Ende des Haltearms befindlichen Drehknopf. Anscheinend wird das Kugelgelenk automatisch verriegelt, wenn der Anwender den Haltearm versehentlich loslässt.

Dieser bekannte Haltearm hat insbesondere die folgenden Nachteile:
- Wie beim Haltearm von Mizuho OZI ist wegen des Abriebs der Kugelfläche ein regelmäßiges Nachstellen des Klemmmechanismus erforderlich;
- Ebenso hat man das Problem mit der Verschmutzung der Kugeloberfläche;
- Im Einsatz am Operationstisch beeinträchtigen das Kugelgelenk und der Haltearm die freie Positionierung eines C-Bogens und die Bildgebung beim Röntgen des Patienten, weil diese im Weg sind und nicht für Röntgenstrahlung durchlässig sind.

Dementsprechend ist es wünschenswert, diese bekannten Haltearme dergestalt zu verbessern, dass sie sich sicherer, wartungsärmer und einfacher ver- und entriegeln lassen.

Es ist also eine Aufgabe der vorliegenden Erfindung, einen medizinischen Haltearm mit arretierbarem Drehgelenk bereitzustellen, bei dem sich das Drehgelenk im täglichen Einsatz zuverlässig, wartungsarm und einfach ver- und entriegeln lässt.

### Überblick über die Erfindung

Erfindungsgemäß wird diese Aufgabe bei dem eingangs definierten Haltearm mit den kennzeichnenden Merkmalen des Anspruchs 1 gelöst.

Durch die Ausgestaltung des Drehgelenks als einen von einer Kipp- und Klemmschelle umgriffenen Zentralkörper mit getrennter Kipp- und Schwenkeinrichtung wird es möglich, eine große Klemmkraft gezielt auf das Drehgelenk aufzubringen.

Bei den bekannten Lösungen mit Kugelgelenk beruht die Klemmung in alle Richtungen ausschließlich auf dem zwischen der Kugel und der Kugelpfanne erzeugten Reibwiderstand und ist direkt abhängig von deren Durchmesser. Dieser Reibwiderstand wird mit der Zeit durch Abnutzung der Kugel und Verschmutzung der Kugeloberfläche immer unzuverlässiger.

Bei der vorliegenden Erfindung hingegen beruht die Klemmung auf zwei getrennten Komponenten: einerseits dem Reibwiderstand zwischen Ringführungsfläche des Zentralkörpers und Führungsring und anderseits dem Reibwiderstand zwischen zylindrische Lauffläche des Zentralkörpers und Kippelement/Klemmschelle. Bei der vorliegenden Erfindung kann der Führungsring klein gehalten werden, da in der Schwenkbewegung niedrige Drehmomente zu erwarten sind. Die wesentlich höheren Drehmomente in Kipprichtung werden von der als Kippelement fungierenden Klemmschelle aufgenommen, die einen entsprechend angepassten, insbesondere größeren Durchmesser aufweist. Die Klemmschelle muss im geklemmten Zustand also nur Drehmomenten in Kipprichtung widerstehen und nicht Drehmomente jeglicher Art abfangen, die auf den Haltearm einwirken können, wie es bei der aus dem obigen Stand der Technik bekannten Kugelpfanne der Fall ist.

Zudem sind bei der vorliegenden Erfindung durch den beanspruchten Aufbau die Reibungsflächen des Drehgelenks weniger Verschleiß ausgesetzt. Da diese außerdem gegenüber der Umwelt abgekapselt sind, können sie nicht verschmutzen. Dementsprechend sind das Risiko eines Ausfalls des Drehgelenks und der einhergehende Wartungsaufwand deutlich reduziert.

Insbesondere können die Reibflächen ölgeschmiert sein, wodurch kein nennenswerter Verschleiß entsteht. Des Weiteren können sich die Reibflächen vollständig umschließen, wodurch kein Schmutz eingetragen oder Schmiermittel ausgetragen werden kann.

Bevorzugte Ausgestaltungen der Erfindung sind Gegenstand der Unteransprüche. Dabei sind sämtliche in den Unteransprüchen aufgeführten Merkmale soweit technisch möglich beliebig miteinander kombinierbar.

Die Erfindung betrifft ebenfalls einen Operationstisch mit einem in den Ansprüchen definierten medizinischen Haltearm.

### Kurze Beschreibung der Zeichnungen

Beispielhafte Ausführungsformen der vorliegenden Erfindung werden nachstehend unter Bezugnahme auf die angehängten Zeichnungen beschrieben, in welchen gleiche Bezugszeichen jeweils gleiche oder einander entsprechende Elemente bezeichnen.
- Fig. 1: zeigt eine Darstellung eines erfindungsgemäßen Operationstisches;
- Fig. 2: zeigt einen medizinischen Haltearm mit arretierbarem Drehgelenk gemäß einer Ausführungsform der vorliegenden Erfindung;
- Fig. 3: ist eine Detailansicht des Drehgelenks bei dem in Fig. 2 gezeigten Haltearm;
- Fig. 4: zeigt einen Freigabehebel, welcher bei dem in Fig. 2 gezeigten Haltearm verwendet werden kann;
- Fig. 5: zeigt ein Anschlussstück des Haltearms aus Fig. 2;
- Fig. 6: zeigt den Zentralkörper sowie die als Kippelement fungierende Klemmschelle des Drehgelenks aus Fig. 3; und
- Fig. 7, 8: illustrieren den Klemmmechanismus des Haltearms aus Fig. 2.

### Detaillierte Beschreibung

In der folgenden Beschreibung werden unter Bezugnahme auf die Zeichnungen beispielhafte Ausführungsformen der vorliegenden Erfindung beschrieben. Die Zeichnungen sind dabei nicht notwendigerweise maßstabsgetreu, sondern sollen die jeweiligen Merkmale lediglich schematisch illustrieren.

Dabei ist zu beachten, dass die nachstehend beschriebenen Merkmale und Komponenten jeweils miteinander kombiniert werden können, unabhängig davon, ob sie in Zusammenhang mit einer einzigen Ausführungsform beschrieben worden sind. Die Kombination von Merkmalen in den jeweiligen Ausführungsformen dient lediglich der Veranschaulichung des grundsätzlichen Aufbaus und der Funktionsweise der beanspruchten Vorrichtung.

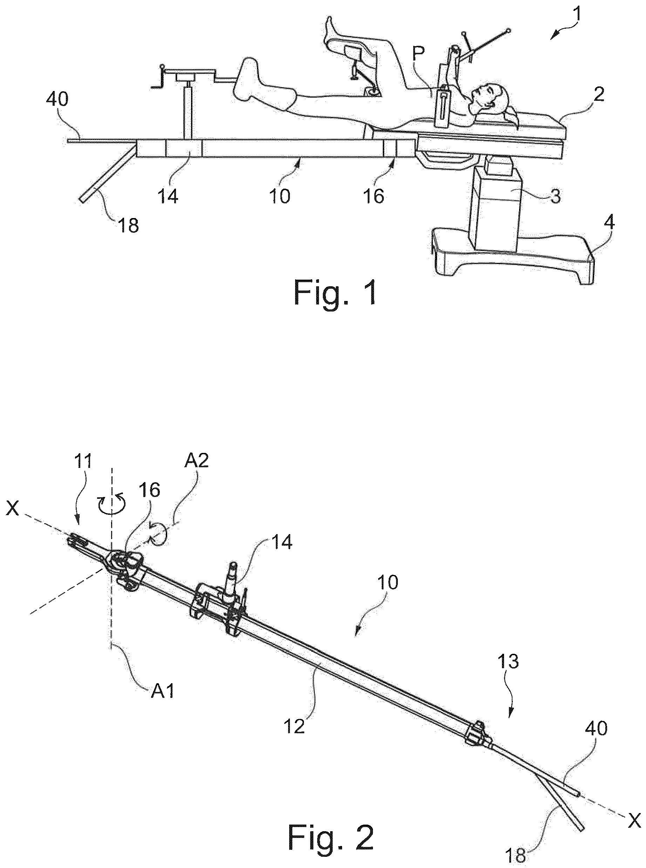

Wie in Fig. 1 dargestellt, umfasst ein Operationstisch 1 eine Patientenlagerfläche 2, eine Säule 3 und einen Fuß 4. Zur Durchführung von orthopädischen Operationen kann ein medizinischer Haltearm 10, in diesem Fall ein Extensionsholm, verwendet werden, mittels dessen das Bein eines Patienten P in einer gewünschten Position fixiert werden kann, wobei sowohl Zugkräfte auf das Bein ausgeübt werden können, als auch das Bein um seine Längsachse verdreht werden kann.

Mittels eines Drehgelenks 16 kann der Extensionsholm 10 relativ zum Operationstisch 1, oder allgemein relativ zu einer Struktur, an der ein Ende des Extensionsholms befestigt ist, im Raum ausgerichtet und fixiert werden.

Hervorzuheben ist, dass sich erfindungsgemäß das Drehgelenk 16 im am Operationstisch 1 montierten Zustand (siehe Fig. 1) möglichst weit in Richtung des Kopfes des Patienten P befindet. Insbesondere sollte sich das Drehgelenk 16 zumindest auf Hüfthöhe oder sogar in Kopfrichtung gesehen oberhalb der Hüfte des Patienten befinden. Dadurch liegt das Drehgelenk außerhalb des röntgentechnisch interessanten Bereichs, wenn mittels eines C-Bogens die Hüfte des Patienten durchleuchtet werden muss. Dabei ist der Extensionsholm 10 bevorzugt aus einem Material gefertigt, welches für die bei der Bildgebung eingesetzte Strahlung transparent ist. Bei der aus der WO 2007/080454 A2 bekannten Lösung befindet sich das Drehgelenk unterhalb des Schritts des Patienten, was die Bildgebung mittels C-Bogens erschwert.

Der in Fig. 2 gezeigte Haltearm 10 umfasst einen Trageholm 12, an dem ein Schlitten 14 angebracht ist. Der Schlitten 14 ist entlang der Längsachse X-X des Trageholms 12 beweglich. Am Schlitten 14 können beispielsweise Halterungen angebracht sein, um einen Patientenfuß einzuspannen (siehe Fig. 1). Bevorzugt besteht der Trageholm 12 aus Material, welches für Röntgenstrahlung durchlässig ist. Auf diese Weise muss der Trageholm 12 beim Röntgen des Patienten P nicht erst aus dem zu röntgenden Bereich entfernt werden, da er in dem späteren Röntgenbild nicht auftaucht. Beispielsweise kann der Trageholm 12 aus Kohlefaserverbundstoff bestehen.

An einem ersten Kopplungsende 11 des Haltearms 10 ist ein Drehgelenk 16 angebracht, mittels dessen der Haltearm 10 relativ zum Operationstisch 1 um zwei Achsen drehbar ist. Das Drehgelenk 16 ist somit ein kardanisches Gelenk, das eine Schwenkachse A1 definiert, um welche der Haltearm 10 in der Horizontalen verschwenkt werden kann, sowie eine Kippachse A2, um welche der Haltearm 10 in der Vertikalen gekippt werden kann.

An einem zweiten Halteende 13 des Haltearms 10 ist ein Freigabehebel 18 angebracht, mittels dessen das Drehgelenk 16 arretiert oder freigegeben werden kann. Zusätzlich zum Freigabehebel 18 ist ein Haltegriff 40 vorgesehen. Somit kann ein Benutzer am Fußende des Patienten stehen, mit einer Hand den Haltegriff 40 ergreifen und mit der anderen Hand den Freigabehebel 18 nach oben ziehen und dort halten, um das Drehgelenk 16 freizugeben, damit er das Patientenbein in eine gewünschten Stellung im Raum positionieren kann. Wenn die gewünschte Position erreicht ist, kann der Benutzer durch Loslassen des Freigabehebels 18, wie nachstehend beschrieben, das Drehgelenk 16 wieder arretieren.

Der Freigabehebel 18 wird bei seinem Anheben um die Kippachse A2 nach oben gedreht.

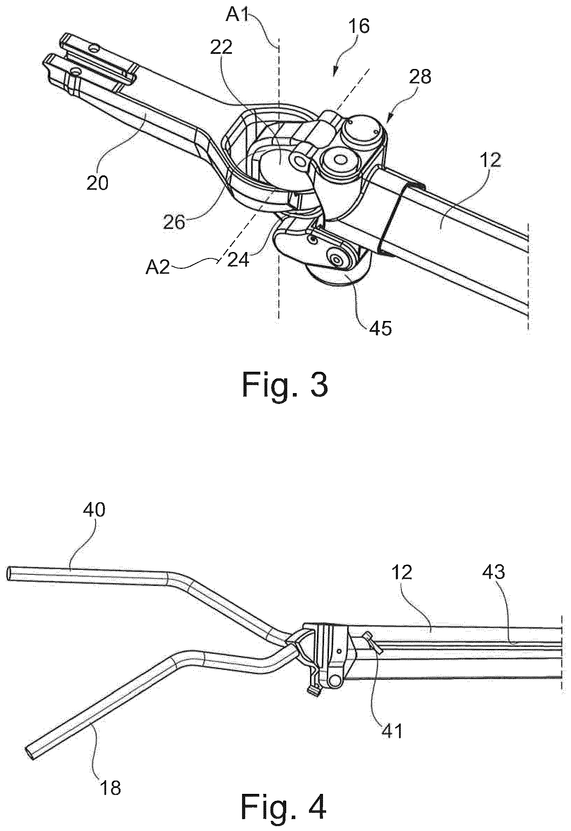

Fig. 3, 5 und 6 zeigen das Drehgelenk 16, sowie ein Anschlussstück 20, das beispielsweise über eine Steckverbindung oder dergleichen mit dem Operationstisch 1 verbunden sein kann. Hierzu verfügt das Anschlussstück 20 über eine Kupplung 21.

Das Drehgelenk 16 umfasst einen Führungsring 36, einen Zentralkörper 22, 24 und eine Klemmschelle 26. Der Zentralkörper besteht aus zwei zueinander spiegelbildlich angeordneten Führungskörpern 22, 24. Die Führungskörper 22, 24 begrenzen zwischen sich einen Hohlraum 33 zur Aufnahme des Führungsrings 36.

Der Führungsring 36 ist drehbar um die Schwenkachse A1 zwischen den zwei Führungskörpern 22, 24 aufgenommen. Er liegt mit seinen beiden Planflächen an den korrespondierenden Ringführungsflächen 30, 32 an. Der Führungsring 36 ist zwischen den Zinken 37 eines gabelförmigen Endes 39 des Anschlussstücks 20 aufgenommen.

Die Klemmschelle 26, welche fest mit dem Trageholm 12 verbunden ist, ist drehbar um die Kippachse A2 auf einer zylindrischen Lauffläche 22a, 24a des Zentralkörpers 22, 24 angebracht. Die Klemmschelle 26 und die zylindrische Lauffläche 22a, 24a bilden gemeinsam eine Kippeinrichtung zum Kippen des Haltearms 10 um die Kippachse A2. Demnach bildet die Klemmschelle 26 ein Kippelement.

Als Teil eines Klemmmechanismus 28 kann die Klemmschelle 26 derart um die Führungskörper 22, 24 herum festgezogen werden, dass sowohl eine Drehung der Klemmschelle 26 relativ zu den Führungskörpern 22, 24, als auch eine Drehung des Führungsrings 36 relativ zu den Führungskörpern 22, 24 verhindert wird.

Der Druck, der von der Klemmschelle 26 auf die beiden Führungskörper 22, 24 ausgeübt wird, üben diese in gleicher Form auf die Planflächen des Führungsrings 36 aus.

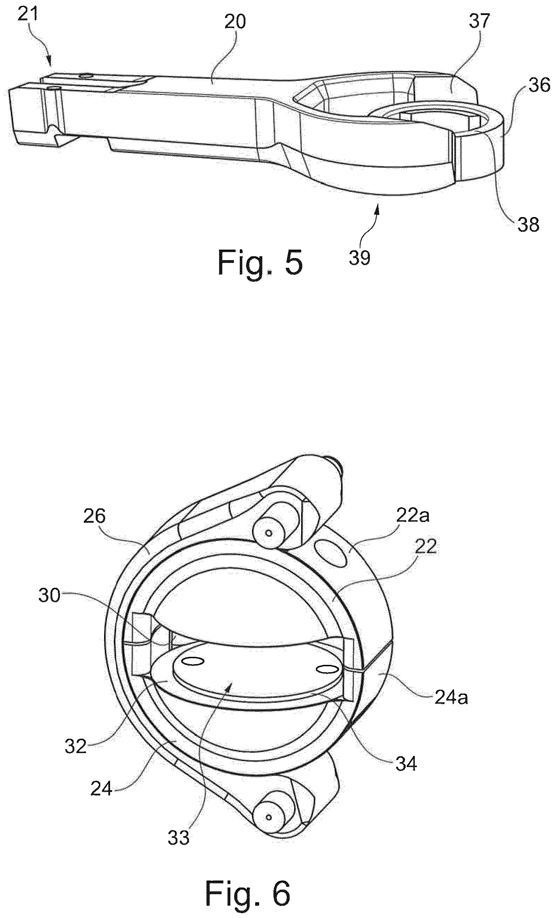

Wie insbesondere aus Fig. 6 ersichtlich ist, werden durch die Klemmschelle 26 die beiden Führungskörper 22, 24 gegeneinander und mittels jeweiliger Kontaktflächen in Form von Ringführungsflächen 30, 32 zwischen den Führungskörpern 22, 24 auf den Führungsring 36 (in Fig. 6 nicht gezeigt) gedrückt, so dass das Drehgelenk 16 in beiden Drehachsen A1, A2 fixiert ist.

Die Ringführungsflächen 30, 32 bilden gemeinsam mit dem Führungsring 36 eine Schwenkeinrichtung zum Verschwenken des Haltearms 10 um die Schwenkachse A1.

Weiterhin ist in Fig. 6 erkennbar, dass die Ringführungsflächen 30, 32 der Führungskörper 22, 24 mit einem oder mehreren kreisbogenförmigen Führungsvorsprüngen 34 ausgestaltet sein können, um die Drehbewegung des Führungsrings 36 um die Schwenkachse A1 zwischen den Führungskörpern 22, 24 zu führen. In Fig. 6 ist ein einziger, kreisförmiger Führungsvorsprung 34 gezeigt, es ist jedoch auch denkbar, mehrere Führungsvorsprünge 34 vorzusehen, welche jeweils einem Kreisbogenabschnitt entsprechen.

Wie in Fig. 5 gezeigt, umfasst das Anschlussstück 20 den Führungsring 36, dessen jeweilige Endflächen 38 als Kontaktflächen zwischen dem Anschlussstück 20 und den Führungskörpern 22, 24 dienen, und in dessen Innenkante der bzw. die Führungsvorsprünge 34 der Führungskörper 22, 24 eingreifen.

Alternativ können Führungsvorsprünge oder andere Führungsmittel auch an den Kontaktflächen 38 des Führungsrings 36 vorgesehen sein. Die Kontaktflächen 38 können in einer weiteren Ausführungsform auch kegelig oder ballig ausgeführt sein. Dabei sollten sie unter einer axial zur Schwenkachse aufgebrachten Kraft zu einer Verspannung führen.

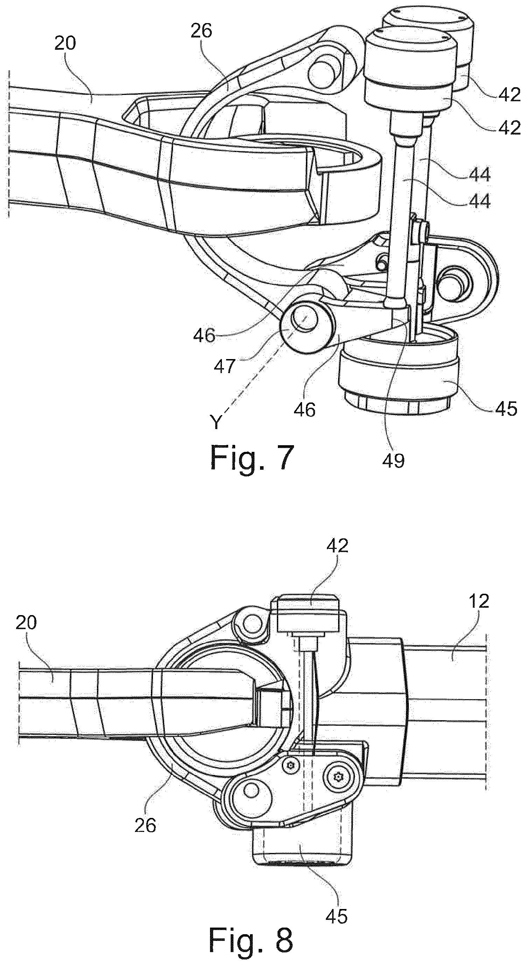

In Fig. 4 ist der Freigabehebel 18 dargestellt. Bei der vorliegenden Ausführungsform ist ein Hebel- bzw. Griffpaar vorgesehen, wobei ein Benutzer den beweglichen Freigabehebel 18 an einen starren Haltegriff 40 heran ziehen kann. Dadurch kann der Benutzer das Gewicht des Holms 12 und eines beispielsweise daran befestigten Patientenbeins mittels des starren Haltegriffs 40 abstützen und bewegen, und dabei gleichzeitig mit dem Freigabehebel 18 den Klemmmechanismus 28 öffnen. Wenn der bewegliche Freigabehebel 18 bis zum Anschlag angehoben ist, liegt die Last des Patientenbeins in beiden Händen des Anwenders, was die Gefahr des ungewollten Absinkens minimiert. Dies trägt zur sicheren Anwendung bei. Bei einer derartigen Betätigung wird Hydraulikflüssigkeit von einem Geberzylinder 41, der in unmittelbarer Nähe des Freigabehebels 18 angebracht ist, über eine im Holm 12 verlaufende Hydraulikleitung 43 zu einem Nehmerzylinder 45 (siehe Fig. 7) geführt, so dass der Klemmmechanismus 28 von seiner arretierten Grundstellung in eine Freigabestellung gebracht wird. Die beiden Hydraulikzylinder 41, 45 bilden mitsamt der Hydraulikleitung 43 gemeinsam eine Kraftübertragungseinrichtung.

Bei der in Fig. 7 gezeigten arretierten Grundstellung drücken Tellerfederpakete 42 mittels Druckstangen 44 zugeordnete Exzenterhebel 46 in eine Position, in der sie eine Extremität der Klemmschelle 26 in eine tangentiale Richtung ziehen und so die Klemmschelle 26 derart auf die zylinderförmige Lauffläche der Führungskörper 22, 24 gedrückt wird, dass die Bewegung des Drehgelenks 16 um beide Drehachsen A1, A2 arretiert ist.

Somit umfasst der Klemmmechanismus 28 die Klemmschelle 26, die Tellerfederpakete 42, die Druckstangen 44, sowie die Exzenterhebel 46. Die zwei Exzenterhebel 46 sind beiderseits der Klemmschelle 26 angeordnet. Sie sind im vorliegenden Fall durch einen Steg verbunden, da sie gemeinsam die gleiche Bewegung ausführen müssen. Die beiden Exzenterhebel 46 bilden mit dem Verbindungsteg ein einheitliches Bauteil.

Jeder Exzenterhebel 46 hat ein Exzenterende 47 in Form einer Exzenterscheibe und ein Betätigungsende 49. Die Drehachse Y-Y der Exzenterhebel 46 verläuft durch eine Extremität der Klemmschelle 26. Die Exzenter-Drehachse Y-Y ist parallel zur Kippachse A2.

Somit wird die Klemmschelle 26 durch die schwenkbaren Exzenterhebel 46 betätigt, um eine Kraftübersetzung in Richtung der Klemmschelle 26 zu generieren. Am langen Hebel des Exzenters 46 übt über die zugeordnete Druckstange 44 das Tellerfederpaket 42 Druckkraft aus und erzeugt damit im Ruhezustand die erforderliche Schließkraft.

Bei Betätigung des Freigabehebels 18 wird der Klemmmechanismus 28 aus der in Fig. 7 und 8 gezeigten arretierten Grundstellung in eine Freigabestellung überführt, in der eine Bewegung des Anschlussstücks 20 um die Schwenkachse A1 und eine Bewegung der Klemmschelle 26 um die Kippachse A2 relativ zu den Führungskörpern 22, 24 (siehe Fig. 3) ermöglicht wird, indem die Exzenterhebel 46 gegen die Federkraft der Tellerfederpakete 42 bewegt werden, so dass der Druck der Klemmschelle 26 auf die Mantelflächen der Führungskörper 22, 24 abgebaut wird, und somit die Klemmschelle 26 und das Anschlussstück 20 relativ zu den Führungskörpern 22, 24 bewegbar sind.

Der Hydrauliknehmerzylinder 45, dessen Wirkrichtung der Federkraft entgegengerichtet ist, ist über die im Holm 12 verlaufende Hydraulikleitung 43 mit dem Geberzylinder 41 verbunden, auf den der Anwender am anderen Ende des Holmes 12 über den Freigabehebel 18 einwirken kann.

Betätigt der Anwender den Freigabehebel 18, so wird im Geberzylinder Flüssigkeit verdrängt und zum Nehmerzylinder gefördert. Dieser arbeitet gegen die Tellerfederpakete 42 an und entlastet die Klemmschelle. Die Klemmschelle 26 öffnet sich und die Bewegung um die beiden Drehachsen A1, A2 wird möglich. Lässt der Bediener den Freigabehebel 18 los, so drücken die Federpakete 42 den Nehmerzylinder zurück und wirken mit ihrer Kraft wieder auf die Klemmschelle 26 ein, das System ist verriegelt.

Die vorliegende Erfindung bietet somit einen Drehmechanismus um zwei aufeinander senkrecht stehende Achsen A1, A2, die durch einen Klemmmechanismus 28 gleichzeitig geklemmt werden, und ein Verriegelungsprinzip, bei dem die Schließkraft von Federpaketen aufgebracht wird und der Bediener nur zum Lösen eine Kraft aufbringen muss. Lässt der Bediener den Freigabehebel 18 los, so fällt das Drehgelenk 16 automatisch in die sichere verriegelte Position zurück. Dadurch kann vermieden werden, dass beispielsweise ein Extensionsholm mit einem Patientenbein unkontrolliert nach unten fällt, wenn der Bediener den Extensionsholm versehentlich loslässt.

Bei der hier dargestellten Ausführungsform werden für das Drehgelenk zwei Führungskörper verwendet, die auf den horizontalen Planflächen 38 des Führungsrings 36 aufliegen und um eine vertikale Achse verschwenkt werden können. Die Außenflächen der beiden Führungskörper bilden eine gemeinsame Zylindermantelfläche, deren Achse A2 horizontal und in Grundstellung senkrecht zur Patientenlängsachse ausgerichtet ist. Diese gemeinsame Zylindermantelfläche wird von einer Klemmschelle 26 umfasst, die mit dem Holm 12 verbunden ist und im ungespannten Zustand eine Rotationsbewegung des Holmes um die Zylindermantelfläche zulässt. Wird diese Klemmschelle 26 nun zugezogen, so wird nicht nur die Normalkraft zwischen zylindrischer Außenfläche 22a, 24a der Führungskörper 22, 24 und Klemmschelle 26 erhöht, sondern auch die Normalkraft zwischen den Planflächen zwischen den Führungskörpern 22, 24 und dem Führungsring 36. Auf diese Art können beide Bewegungen mit einer Betätigung arretiert und gelöst werden.

Es ist jedoch auch denkbar, dass anstelle der Führungskörper anders geformte Gelenkelemente verwendet werden können, solange damit ein Gelenk realisiert werden kann, welches um mindestens zwei Drehachsen drehbar ist, und solange damit ein Klemmmechanismus realisiert werden kann, durch welchen die Bewegung des Gelenks in beiden Achsen gleichzeitig arretiert werden kann.

Der erfindungsgemäße medizinische Haltearm hat dabei den Vorteil, dass eine sichere Bedienung gewährleistet werden kann, denn wenn ein Anwender versehentlich den Haltearm mit oder ohne einem darauf befestigten Patientenbein fallen lässt, wird das Drehgelenk, das eine Bewegung des Haltearms relativ zu einem Operationstisch ermöglicht, automatisch arretiert.

Somit kann der Haltearm nicht unkontrolliert zu Boden fallen. Durch die am Fußende des Patienten angebrachten Freigabehebel 18 und Haltegriff 40 kann der Benutzer den Haltearm bei der Verstellung mit beiden Händen führen und abstützen, so dass eine sichere und ergonomische Verstellung der Position des Haltearms 10 möglich ist.

Der beschriebene Klemmmechanismus 28, im Zusammenspiel mit der gewählten Ausgestaltung des Drehgelenks 16 sowie des Halteendes 13 des Haltearms 10, erlaubt eine sichere Verriegelung des Drehgelenks 16, trotz der großen Kräfte und Drehmomente, die wegen der großen Länge des Haltearms 10 auf das Drehgelenk einwirken. Mit der aus der WO 2007/080454 A2 bekannten Verriegelung, bei der ein einfacher Handgriff 118 und ein Kugelgelenk verwendet wird, lassen sich die hohen Drehmomente, die dadurch auftreten, dass der hier beschriebene lange Haltearm 10 jenseits der Patientenhüfte am Operationstisch 10 angebunden ist, nicht zuverlässig auffangen. Bei der hier beschriebenen erfinderischen Lösung werden die erforderlichen hohen Klemmkräfte insbesondere durch die Hydraulik und die große Übersetzung (z.B. im Verhältnis 1:10) der Exzenterhebel 46 ermöglicht. Dank des großen Hebelarms des Freigabehebels 18 kann der Anwender die hohen Klemmkräfte mit geringem Kraftaufwand überwinden.

## Patentansprüche

1. Medizinischer Haltearm (10) umfassend:
- ein Halteende (13) zum Ergreifen und Bedienen des Haltearms (10);
- ein Kopplungsende (11) zum Anschließen des Haltearms an eine Patientenaufnahme wie z.B. einen Operationstisch (1);
- ein am Kopplungsende befindliches Drehgelenk (16) zum Schwenken des Haltearms um eine Schwenkachse (A1) und zum Kippen des Haltearms um eine Kippachse (A2), die sich mit der Schwenkachse schneidet;
- einen Klemmmechanismus (28), mit dem das Drehgelenk wahlweise:
a) verriegelt werden kann, sodass ein Drehen des Haltearms (10) sowohl um die Schwenk- als auch die Kippachse unterbunden ist;
b) gelöst werden kann, sodass sich der Haltearm um beide Achsen (A1, A2) frei drehen lässt;
- einen am Halteende (13) befindlichen Freigabehebel (18) zum Lösen des Klemmmechanismus (28);
- einen Trageholm (12) zum Tragen eines medizinischen Geräts (14), der sich zwischen dem Halteende (13) und dem Kopplungsende (11) erstreckt; und
- eine Kraftübertragungseinrichtung (41, 43, 45) zur Übertragung einer Lösekraft vom Freigabehebel (18) zum Klemmmechanismus, die im und/oder am Trageholm angeordnet ist,
**dadurch gekennzeichnet, dass**
- das Drehgelenk (16):
a) ein Drehen des Haltearms (10) ausschließlich um die Kippachse (A2) und die Schwenkachse (A1) zulässt;
b) eine Kippeinrichtung zum Kippen des Haltearms (10) um die Kippachse (A2) umfasst, wobei die Kippeinrichtung ein Kippelement (26) und eine zylindrische Lauffläche (22a, 24a) aufweist, auf welcher das Kippelement sitzt, sodass sich die zylindrische Lauffläche und das Kippelement relativ zueinander um die Kippachse (A2) verdrehen können;
c) eine Schwenkeinrichtung zum Verschwenken des Haltearms (10) um die Schwenkachse (A1) umfasst, wobei die Schwenkeinrichtung einen Führungsring (36) und zumindest eine den Führungsring aufnehmende Ringführungsfläche (30, 32) aufweist, sodass sich der Führungsring und die wenigstens eine Ringführungsfläche relativ zueinander um die Schwenkachse (A1) verdrehen können; und
d) einen Zentralkörper (22, 24) umfasst, der sich im Zentrum des Drehgelenks befindet und an dem die zylindrische Lauffläche (22a, 24a) und die wenigstens eine Ringführungsfläche (30, 32) ausgebildet sind;
- das Kippelement (26) als Teil des Klemmmechanismus ebenfalls derart als Klemmschelle fungiert, dass diese durch Einklemmen des Zentralkörpers (22, 24) sowohl die Kippeinrichtung als auch die Schwenkeinrichtung gegen ein Verdrehen verklemmt.

2. Medizinischer Haltearm (10) nach Anspruch 1, wobei die zylindrische Lauffläche (22a, 24a) einmal vollständig um die Kippachse (A2) verläuft.

3. Medizinischer Haltearm (10) nach einem der vorherigen Ansprüche, wobei der Zentralkörper zwei zueinander spiegelbildlich angeordnete Führungskörper (22, 24) umfasst, wobei in jedem Führungskörper (22, 24) eine Ringführungsfläche (30, 32) für den Führungsring ausgebildet ist, und wobei an der Außenseite eines jeden Führungskörpers ein Teil (22a, 24a) der zylindrischen Lauffläche ausgebildet ist, sodass die beiden Teile gemeinsam die gesamte zylindrische Lauffläche bilden.

4. Medizinischer Haltearm (10) nach einem der vorherigen Ansprüche, wobei der Zentralkörper in seinem Innern einen Hohlraum (33) zur Aufnahme des Führungsrings aufweist.

5. Medizinischer Haltearm (10) nach einem der vorherigen Ansprüche, zusätzlich mit einem Anschlussstück (20) am Kopplungsende (11), wobei das Anschlussstück den Führungsring (36) trägt und über eine Kupplung (21) zum Anschluss an die Patientenaufnahme verfügt, wobei vorzugsweise der Führungsring (36) zwischen den Zinken (37) eines gabelförmigen Endes (39) des Anschlussstücks aufgenommen ist.

6. Medizinischer Haltearm (10) nach einem der vorherigen Ansprüche, wobei der Klemmmechanismus (28) eine Federanordnung (42) umfasst, welche die Klemmschelle in Verriegelungsstellung vorspannt.

7. Medizinischer Haltearm (10) nach Anspruch 6, wobei der Klemmmechanismus (28) außerdem eine Exzenteranordnung (46) umfasst, über welche die Federanordnung auf die Klemmschelle einwirkt.

8. Medizinischer Haltearm (10) nach Anspruch 7, wobei die Federanordnung (42) mindestens eine Tellerfeder umfasst, und wobei die Exzenteranordnung (46) wenigstens einen Exzenterhebel umfasst.

9. Medizinischer Haltearm (10) nach Anspruch 8, wobei jeder Exzenterhebel (46) ein Exzenterende in Form einer Exzenterscheibe (47) und ein Betätigungsende (49) hat, wobei das Betätigungsende mit einer Tellerfeder (42) verbunden ist, und wobei das Exzenterende (47) mit einer Extremität der Klemmschelle (26) verbunden ist, durch welche die Drehachse (Y-Y) des Exzenterhebels verläuft.

10. Medizinischer Haltearm (10) nach einem der vorherigen Ansprüche, wobei die Kraftübertragungseinrichtung folgendes umfasst:
- einen am Halteende angeordneten Geberzylinder (41);
- einen am Kopplungsende angeordneten Nehmerzylinder (45); und
- eine den Geberzylinder mit dem Nehmerzylinder hydraulisch verbindende Hydraulikleitung (43), die durch das Innere des Trageholms verläuft.

11. Medizinischer Haltearm (10) nach Anspruch 10 in Kombination mit einem der Ansprüche 6 bis 9, wobei der Nehmerzylinder (45) bei Betätigung des Freigabehebels hydraulisch die Vorspannung der Federanordnung löst.

12. Medizinischer Haltearm (10) nach einem der vorherigen Ansprüche, wobei die Schwenkachse (A1) und die Kippachse (A2) jeweils senkrecht zu einer Längsachse (X-X) des medizinischen Haltearms (10) und senkrecht zueinander ausgerichtet sind.

13. Medizinischer Haltearm (10) nach einem der vorherigen Ansprüche, wobei der medizinische Haltearm ein Extensionsholm (10) ist.

14. Medizinischer Haltearm (10) nach einem der vorherigen Ansprüche, weiterhin mit einem am Halteende befindlichen Haltegriff (40), der derart neben dem Freigabehebel (18) angeordnet ist, dass der medizinische Haltearm (10) insbesondere bei gelöstem Klemmmechanismus (28) von einer Person mit der einen Hand am Freigabehebel (18) und der anderen Hand am Haltegriff (40) gehalten werden kann.

15. Operationstisch (1) umfassend einen medizinischen Haltearm nach einem der vorherigen Ansprüche.

## Claims

1. A medical support arm (10) comprising:
- a holding end (13) for gripping and operating the support arm (10);
- a coupling end (11) for connecting the support arm to a patient receptacle such as e.g. an operating table (1);
- a rotary joint (16) located at the coupling end for pivoting the support arm about a pivot axis (A1) and for tilting the support arm about a tilting axis (A2) which intersects with the pivot axis;
- a clamping mechanism (28) with which the rotary joint optionally:
a) can be locked so that rotation of the support arm (10) about both the pivoting and the tilting axis is prevented;
b) can be released so that the support arm can rotate about both axes (A1, A2) freely;
- a release lever (18) located at the holding end (13) for releasing the clamping mechanism (28);
- a support bar (12) for supporting a medical device (14), which support bar extends between the holding end (13) and the coupling end (11); and
- a force transmission device (41, 43, 45) for transmitting a release force from the release lever (18) to the clamping mechanism, which force transmission device is arranged in and/or on the support bar,
**characterized in that**
- the rotary joint (16):
a) permits rotating the support arm (10) only about the tilting axis (A2) and the pivot axis (A1);
b) comprises a tilting device for tilting the support arm (10) about the tilting axis (A2), wherein the tilting device comprises a tilting element (26) and a cylindrical running surface (22a, 24a) on which the tilting element sits, so that the cylindrical running surface and the tilting element can rotate relative to one another around the tilting axis (A2);
c) comprises a pivoting device for pivoting the support arm (10) about the pivot axis (A1), wherein the pivoting device comprises a guide ring (36) and at least one ring guide surface (30, 32) receiving the guide ring so that the guide ring and the at least one ring guide surface can rotate relative to one another about the pivot axis (A1); and
d) comprises a central body (22, 24) located at the center of the rotary joint and on which the cylindrical running surface (22a, 24a) and the at least one ring guide surface (30, 32) are formed;
- the tilting element (26) as part of the clamping mechanism also acts as a collar in such a way that by pinching the central body (22, 24), this clamps both the tilting device and the pivoting device against rotation.

2. The medical support arm (10) of claim 1, wherein the cylindrical running surface (22a, 24a) runs completely around the tilting axis (A2).

3. The medical support arm (10) of any one of the preceding claims, wherein the central body comprises two guide bodies (22, 24) arranged mirror-inverted to each other, wherein a ring guide surface (30, 32) for the guide ring is formed in each guide body (22, 24), and wherein on the outside of each guide body a part (22a, 24a) of the cylindrical running surface is designed so that the two parts together form the entire cylindrical running surface.

4. The medical support arm (10) of any one of the preceding claims, wherein the central body has in its interior a cavity (33) for receiving the guide ring.

5. The medical support arm (10) of any one of the preceding claims, additionally having a connector (20) on the coupling end (11), wherein the connector bears the guide ring (36) and has a coupling (21) for connection to the patient receptacle, wherein preferably the guide ring (36) is received between the prongs (37) of a forked end (39) of the connector.

6. The medical support arm (10) of any one of the preceding claims, wherein the clamping mechanism (28) comprises a spring assembly (42) which biases the collar in locking position.

7. The medical support arm (10) of claim 6, wherein the clamping mechanism (28) also comprises an eccentric arrangement (46) via which the spring assembly acts on the collar.

8. The medical support arm (10) of claim 7, wherein the spring assembly (42) comprises at least one disc spring, and wherein the eccentric arrangement (46) comprises at least one eccentric lever.

9. The medical support arm (10) of claim 8, wherein each eccentric lever (46) has an eccentric end in the form of an eccentric disk (47) and an actuating end (49), wherein the actuating end is connected to a plate spring (42), and wherein the eccentric end (47) is connected to an extremity of the collar (26) through which the axis of rotation (Y-Y) of the eccentric lever runs.

10. The medical support arm (10) of any one of the preceding claims, wherein the force transmission device includes:
- a driving cylinder (41) arranged at the holding end;
- a driven cylinder (45) arranged at the coupling end; and
- a hydraulic line (43) that runs through the inside of the supporting bar and connects the driving cylinder hydraulically to the driven cylinder.

11. The medical support arm (10) of claim 10 in combination with one of claims 6 to 9, wherein the driven cylinder (45) hydraulically releases the biasing of the spring assembly when the release lever is actuated.

12. The medical support arm (10) of any one of the preceding claims, wherein the pivot axis (A1) and the tilting axis (A2) are each perpendicular to a longitudinal axis (X-X) of the medical support arm (10) and are aligned perpendicular to each other.

13. The medical support arm (10) of any one of the preceding claims, wherein the medical support arm is an extension bar (10) .

14. The medical support arm (10) of any one of the preceding claims, additionally having a handle (40) located on the holding end, which is arranged in such a way adjacent to the release lever (18) that the medical support arm (10), in particular when the clamping mechanism (28) is released, can be held by a person with one hand on the release lever (18) and the other hand on the handle (40).

15. An operating table (1) comprising a medical support arm of any one of the preceding claims.

## Revendications

1. Bras de support médical (10) comprenant :
- une extrémité de maintien (13) pour saisir et manœuvrer le bras de maintien (10) ;
- une extrémité de couplage (11) pour raccorder le bras de support à un logement de patient tel que par exemple une table d'opération (1) ;
- une articulation tournante (16) se trouvant à l'extrémité de couplage pour faire pivoter le bras de support autour d'un axe de pivotement (A1) et pour incliner le bras de support autour d'un axe d'inclinaison (A2) qui coupe l'axe de pivot ;
- un mécanisme de serrage (28) avec lequel l'articulation tournante au choix :
a) peut-être verrouillé de sorte que la rotation du bras de support (10) est empêchée aussi bien autour de l'axe de pivotement que de l'axe de basculement ;
b) peut-être libéré de sorte que le bras de maintien puisse tourner librement autour des deux axes (A1, A2) ;
- un levier de déverrouillage (18) situé à l'extrémité de maintien (13) pour libérer le mécanisme de serrage (28) ;
- une barre de support (12) pour porter un dispositif médical (14) qui s'étend entre l'extrémité de maintien (13) et l'extrémité de couplage (11) ; et
- un dispositif de transmission de force (41, 43, 45) pour transmettre une force de déclenchement depuis le levier de déverrouillage (18) vers le mécanisme de serrage, qui est disposé dans et/ou sur la barre de support,
**caractérisé en ce que**
- l'articulation tournante (16) :
a) permet une rotation du bras de support (10) exclusivement autour de l'axe d'inclination (A2) et de l'axe de pivotement (A1) ;
b) comprend un dispositif d'inclinaison pour incliner le bras de support (10) autour de l'axe d'inclinaison (A2), dans lequel le dispositif d'inclinaison comprend un élément d'inclinaison (26) et une surface de roulement cylindrique (22a, 24a) sur lequel repose l'élément d'inclinaison, de sorte que le cylindre peut faire pivoter la surface de roulement et l'élément d'inclinaison l'un par rapport à l'autre autour de l'axe d'inclinaison (A2) ;
c) comprend un dispositif de pivotement pour faire pivoter le bras de maintien (10) vers l'axe de pivotement (A1), dans lequel le dispositif de pivotement comprend une bague de guidage (36) et au moins une surface de guidage de bague (30, 32) recevant la bague de guidage, de sorte que la bague de guidage et l'au moins une surface de guidage de bague peuvent tourner l'un par rapport à l'autre autour de l'axe de pivotement (A1) ; et
d) comprend un corps central (22, 24) qui se trouve au centre de l'articulation tournante et sur lequel sont formées la surface de roulement cylindrique (22a, 24a) et l'au moins une surface de guidage de bague (30, 32) ;
- l'élément d'inclinaison (26), en tant que partie du mécanisme de serrage, sert également de collier de serrage de telle sorte qu'en serrant le corps central (22, 24), il bloque à la fois le dispositif d'inclinaison et le dispositif de pivotement contre une rotation.

2. Bras de support médical (10) selon la revendication 1, dans lequel la surface de roulement cylindrique (22a, 24a) tourne complètement autour de l'axe d'inclinaison (A2).

3. Bras de support médical (10) selon l'une des revendications précédentes, dans lequel le corps central comprend deux corps de guidage mutuellement inversés (22, 24), dans lequel, dans chaque corps de guidage (22, 24), une surface de guidage de bague (30, 32) est formée pour la bague de guidage, et dans lequel une partie (22a, 24a) de la surface de roulement cylindrique est formée sur le côté extérieur de chaque corps de guidage de sorte que les deux parties forment ensemble la totalité de la surface de roulement cylindrique.

4. Bras de support médical (10) selon l'une des revendications précédentes, dans lequel le corps central comporte à l'intérieur une cavité (33) de réception de la bague de guidage.

5. Bras de support médical (10) selon l'une des revendications précédentes, comprenant en outre une pièce de raccordement (20) à l'extrémité de couplage (11), dans lequel la pièce d'accordement porte la bague de guidage (36) et dispose d'un couplage (21) pour le raccordement au logement du patient, dans lequel la bague de guidage (36) est de préférence reçu entre les dents (37) d'une extrémité en forme de fourche (39) de la pièce de raccordement.

6. Bras de support médical (10) selon l'une des revendications précédentes, dans lequel le mécanisme de serrage (28) comprend un ensemble de ressort (42) qui sollicite le collier de serrage en position de verrouillage.

7. Bras de support médical (10) selon la revendication 6, dans lequel le mécanisme de serrage (28) comprend également un ensemble excentrique (46) par l'intermédiaire duquel l'ensemble de ressort agit sur le collier de serrage.

8. Bras de support médical (10) selon la revendication 7, dans lequel l'ensemble de ressort (42) comprend au moins un ressort à disque, et dans lequel l'ensemble excentrique (46) comprend au moins un levier excentrique.

9. Bras de support médical (10) selon la revendication 8, dans lequel chaque levier excentrique (46) a une extrémité excentrique sous la forme d'un disque excentrique (47) et une extrémité d'actionnement (49), dans lequel l'extrémité d'actionnement est reliée à un ressort à disque (42), et dans lequel l'extrémité excentrique (47) est reliée à une extrémité du collier de serrage (26) à travers laquelle passe l'axe de rotation (Y-Y) du levier excentrique.

10. Bras de support médical (10) selon l'une des revendications précédentes, dans lequel la transmission de force comprend :
- un maître-cylindre (41) disposé à l'extrémité de maintien ;
- un cylindre récepteur (45) disposé à l'extrémité de couplage ; et
- une conduite hydraulique (43) reliant hydrauliquement le cylindre émetteur au cylindre récepteur, qui s'étend à travers l'intérieur de la barre de support.

11. Bras de maintien médical (10) selon la revendication 10 en combinaison avec l'une des revendications 6 à 9, dans lequel le cylindre récepteur (45), lorsque le levier de déverrouillage est actionné hydrauliquement, résout la précharge de l'assemblage du ressort.

12. Bras de support médical (10) selon l'une des revendications précédentes, dans lequel l'axe de pivotement (A1) et l'axe d'inclinaison (A2) chacun perpendiculaire sont respectivement orientés perpendiculairement à un axe longitudinal (X-X) du bras de support médical (10) et perpendiculairement l'un par rapport à l'autre.

13. Bras de support médical (10) selon l'une des revendications précédentes, dans lequel le bras de support médical est une barre d'extension (10).

14. Bras de support médical (10) selon l'une des revendications précédentes, comprenant en outre une poignée de maintien (40) se trouvant à l'extrémité de maintien, qui est disposée à côté du levier de déverrouillage (18) de telle sorte que le bras de support médical (10), en particulier lorsque le mécanisme de serrage (28) est desserré, peut être tenu par une personne avec une main sur le levier de déverrouillage (18) et l'autre main sur la poignée de maintien (40),

15. Table d'opération (1) comprenant un bras de maintien médical selon l'une des revendications précédentes.
